(19) Europäisches Patentamt
European Patent Office
Office européen des brevets

(11) Numéro de publication : **0 365 387 B1**

(12) # FASCICULE DE BREVET EUROPEEN

(45) Date de publication du fascicule du brevet : **19.11.92 Bulletin 92/47**

(51) Int. Cl.$^5$ : **C07C 31/125,** C07C 29/136

(21) Numéro de dépôt : **89402712.7**

(22) Date de dépôt : **03.10.89**

(54) **Procédé de fabrication d'alcools gras et installation de mise en oeuvre.**

(30) Priorité : **18.10.88 FR 8813074**

(43) Date de publication de la demande : **25.04.90 Bulletin 90/17**

(45) Mention de la délivrance du brevet : **19.11.92 Bulletin 92/47**

(84) Etats contractants désignés : **DE FR GB**

(56) Documents cités :
**FR-A- 1 118 624**
**FR-A- 1 122 860**
**FR-A- 1 157 346**

(73) Titulaire : **L'AIR LIQUIDE, SOCIETE ANONYME POUR L'ETUDE ET L'EXPLOITATION DES PROCEDES GEORGES CLAUDE**
**75, Quai d'Orsay**
**F-75321 Paris Cédex 07 (FR)**

(72) Inventeur : **Dupont, René**
**113, Grande Rue Charles de Gaulle**
**F-94130 Nogent-sur-Marne (FR)**
Inventeur : **Ferenczi, Laurent**
**13, passage Ramey**
**F-75018 Paris (FR)**
Inventeur : **Simonet, Guy**
**105, Cours de Vincennes**
**F-75020 Paris (FR)**

(74) Mandataire : **Le Moenner, Gabriel et al**
**L'AIR LIQUIDE 75, Quai d'Orsay**
**F-75321 Paris Cédex 07 (FR)**

EP 0 365 387 B1

Il est rappelé que : Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

## Description

La présente invention concerne un procédé de production d'alcool gras.

La production d'alcool gras, à partir d'esters méthyliques est mise en oeuvre dans un réacteur d'hydrogénation selon la réaction (A)

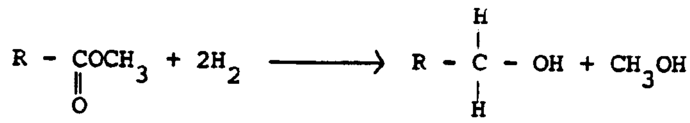

Les alcools gras produits sont ensuite séparés du résiduaire méthanolé, celui-ci étant constitué principalement de méthanol et d'alcools supérieurs.

Puis ce résidu est traité soit par combustion dans une chaudière ou en le brûlant à la torche, soit purifié par distillation quand il y a des besoins en méthanol pur sur le site industriel ou des possibilités de commercialisation du méthanol. Mais, la distillation conduit à des dépenses énergétiques et des pertes de méthanol, principalement dues à l'entraînement dans l'eau en cuve de colonne.

D'autre part, l'hydrogène nécessaire à la réaction est soit produit le site par divers procédés tels que craquage ou reformage d'hydrocarbures, électrolyse, etc... soit de provenance externe.

Les alcools gras, obtenus à partir de matières grasses végétales, sont produits en deux étapes ou selon deux chaînes de production à savoir la transestérification et l'hydrogénation. Selon la figure 1 du dessin annexé, qui représente un schéma fonctionnel, les liquides d'alimentation, le méthanol et les matières grasses, sont introduits par les circuits (1) et (2) vers la transestérification (3) première étape de production des esters méthyliques directement véhiculés par le circuit (4) vers la phase d'hydrogénation (5) alimenté en hydrogène par la conduite (6). Au cours de la seconde étape de production les esters méthyliques en présence d'hydrogène sont transformés selon la réaction (A) en alcool gras recueillis en (7) et le résiduaire méthanol, ou méthanol impur est véhiculé par le circuit (8) jusqu'au stade de rectification par distillation en (9) et le méthanol purifié est recyclé par le circuit (10) à l'alimentation (1) en méthanol.

Sur les sites qui produisent eux-mêmes la totalité des esters méthyliques à hydrogéner, les besoins en méthanol pur sont en principe au moins égaux à la quantité qu'on peut obtenir par distillation du méthanol impur. Cependant, les dépenses énergétiques, les pertes et les pollutions liées à la distillation font envisager la recherche d'une meilleure valorisation du méthanol impur.

En outre, un certain nombre de sites importent les esters à hydrogéner, et leur besoin en méthanol pur devient inférieur à leur production de méthanol impur. Suivant les cours du méthanol, la distillation peut s'avérer non économique, et le produit purifié invendable.

Aussi, il a été recherché un procédé de fabrication d'alcools gras par hydrogenation des esters méthyliques, sans recyclage du méthanol impur sous forme de méthanol pur à l'entrée d'une éventuelle production d'esters.

Il est proposé de conduire la réaction d'hydrogénation des esters méthyliques conjointement à une réaction de vaporeformage de méthanol.

Le méthanol impur issu dans la réaction d'hydrogénation des esters méthyliques est soumis à la réaction de vaporeformage et l'hydrogène ainsi obtenu est recyclé à l'entrée de la chaîne de production d'alcools gras.

Le processus consiste à recycler le méthanol impur non plus sous forme de méthanol pur à l'entrée de la chaîne de production d'esters, mais sous forme d'hydrogène pour la chaîne d'hydrogénation.

Ce recyclage permet de rendre les deux étapes de production d'alcool gras, à partir de matières grasses végétales, indépendantes dans de bonnes conditions économiques.

L'indépendance des deux chaînes de production : transestérification et hydrogénation, donne aux industriels la possibilité de répartir les fonctions de production d'esters et production d'alcools au mieux entre leurs différents sites, dans les pays producteurs d'huiles végétales et les pays consommateurs d'alcools gras.

Le procédé de reformage associé à l'hydrogénation des esters méthyliques est d'un type quelconque de vaporeformage de méthanol impur. Notamment, un reformage basse température et le procédé de reformage de méthanol impur selon la demande de brevet FR 87 15006, au nom de la déposante est bien adapté. Suivant, ce procédé de reformage de méthanol impur, contenant des alcools supérieurs au méthanol, dans lequel l'apport de chaleur se fait par l'intermédiaire d'un fluide caloporteur chauffé par le gaz résiduaire, d'une purification du gaz produit par reformage, en vue de l'obtention d'hydrogène pur, on réalise une combustion simultanée du mélange condensé eau-alcools provenant du refroidissement du gaz reformé, d'une part, et du gaz résiduaire provenant de la purification de l'hydrogène.

Selon une variante, dans le cas où le méthanol contient des impuretés lourdes, après un temps de repos aproprié le mélange : eau + méthanol impur est filtré pour séparer la phase solide, et le liquide filtré alimente l'unité de reformage.

Le procédé selon l'invention peut être mis en oeuvre dans une installation en deux chaînes de production indépendantes du type représenté sur la figure 2.

La chaîne de production A comprend essentiellement un réacteur de transestérification (11) dans lequel s'effectue la réaction du méthanol et des matières grasses introduits en (12) et (13), après transestérification dans les conditions classiques dans cette technique, les esters méthyliques produits sont soutirés par le circuit (14) vers la chaîne de production B.

Cette seconde chaîne de production B comprend essentiellement un réacteur d'hydrogénation (15) alimenté par les esters méthyliques (14) provenant de la chaîne de production A, et en hydrogène pur recyclé (16) provenant du vaporeformage (17), les alcools gras formés lors de l'hydrogénation sont séparés et soutirés en (18) et le méthanol impur est envoyé par le circuit (19) vers le dit vaporeformage (17).

Une étude comparée des dépenses énergétiques de deux possibilités de traitement du méthanol impur met en évidence les avantages du procédé proposé. Cette comparaison est faite sur un site simple, où l'on réalise les deux opérations de transestérification et d'hydrogénation de manière équilibrée.

Le méthanol impur issu des réacteurs d'hydrogénation a une composition variable suivant les esters traités. Le tonnage considéré représente la quantité de méthanol pur contenu dans les mélanges méthanol + impuretés.

Considérons une usine produisant 20.000 t/an d'alcools gras à partir de 22.000 t/an d'esters methyliques. Le besoin en hydrogène est d'environ 250 Nm3 par tonne d'esters traité, soit 5.500.000 Nm3/an. La quantité de méthanol impur dégagée est de 4.500 t/an.

Selon la méthode classique on procède à la distillation du méthanol impur et l'hydrogène est produit par vaporeformage de gaz naturel dont la consommation est de 0,5 Nm3 de gaz naturel par Nm3 d'hydrogène. La dépense énergétique exprimée en tonnes équivalent pétrole ou tep (avec l'équivalence 1 tep = 1100 Nm3 de gaz naturel) est la suivante.

| | |
|---|---|
| Besoins calorifiques de la distillation : | |
| 160 Nm3 de gaz naturel par tonne de méthanol distillé soit : | 650 tep |
| Pertes de méthanol (entraînement dans l'eau en cuve de colonne, pertes par distillation, etc...) 12 % de la charge. | |
| En valorisant le méthanol à son pouvoir calorifique inférieur soit 0,54 tep/tonne, la perte représente : | 290 tep |
| Production d'hydrogène : | 2.500 tep |
| Dépense totale : | 3.440 tep |

Selon l'invention, on procède à la réaction d'hydrogénation conjointement au reformage du méthanol impur. Le reformage du méthanol impur fournit la quantité d'hydrogène nécessaire. Le reformage est autothermique et la seule dépense d'énergie provient des 4.500 t de méthanol neuf qu'il faut introduire à l'entrée de la transestérification. Avec la même valorisation du méthanol on obtient :

Dépense totale : 2.450 tep

L'économie d'énergie est donc d'environ 1 000 tep.

Outre, les économies d'énergie, le procédé permet par rapport à la méthode classique un investissement plus réduit : économie sur la colonne de distillation de méthanol et sur l'unité de production d'hydrogène puisqu'un reformage de méthanol et en général moins coûteux qu'un reformage de gaz naturel (température plus basse et procédé plus simple).

Le procédé est applicable dans l'industrie des alcools gras dits naturels.

Ces alcools sont utilisés dans la formulation de détergents, cosmétiques, additifs, etc...

De façon générale, toute industrie consommatrice d'hydrogène et productrice de méthanol impur ou non, peut utiliser ce procédé.

## Revendications

1. Procédé de fabrication d'alcools gras par hydrogénation des esters méthyliques, caractérisé en ce que la réaction d'hydrogénation des esters méthyliques est conduite conjointement à une réaction de vaporeformage de méthanol.

2. Procédé de fabrication d'alcools gras par hydrogénation des esters méthyliques selon la revendication 1, caractérisé en ce que le méthanol impur produit dans la réaction d'hydrogénation des esters méthyliques est soumis à la réaction de vaporeformage et l'hydrogène ainsi obtenu est recyclé à l'entrée de la chaîne de production d'alcools gras.

3. Procédé de fabrication d'alcool gras par hydrogénation des esters méthyliques selon la revendication 2, caractérisé en ce que dans la mise en oeuvre du procédé de reformage de méthanol impur contenant des alcools supérieurs au méthanol, dans lequel l'apport de chaleur se fait par l'intermédiaire d'un fluide caloporteur chauffé par le gaz résiduaire, d'une purification du gaz produit par reformage, en vue de l'obtention d'hydrogène pur, on réalise une combustion simultanée du mélange condensé eau-alcools provenant du refroidissement du gaz reformé, d'une part, et du gaz résiduaire provenant de la purification de l'hydrogène.

4. Procédé de reformage de méthanol impur, selon la revendication 3, caractérisé en ce qu'après un temps de repos approprié le mélange eau + méthanol impur est filtré, pour séparer la phase solide et le liquide filtré alimente l'unité de reformage.

5. Installation de fabrication d'alcools gras selon une quelconque des revendications 1 à 4, caractérisée en ce qu'elle est constituée par la chaîne de production A comprenant essentiellement un réacteur de transestérification (11) dans lequel s'effectue la réaction du méthanol et des matières grasses introduits en (12) et (13), les esters méthyliques produits étant soutirés par la circuit (14), puis par la chaîne de production B comprenant essentiellement un réacteur d'hydrogénation (15) alimenté par les esters méthyliques (14) et l'hydrogène recyclé (16) provenant du vaporeformage (17), les alcools gras formés après séparation étant soutirés en (18) et le méthanol impur circulant dans la conduite (19) vers le dit vaporeformage.

## Patentansprüche

1. Verfahren zur Herstellung von Fettalkoholen durch Hydrierung der Methylester, **dadurch gekennzeichnet**, daß die Hydrierreaktion der Methylester gemeinsam mit einer Methanol-Dampfreformierungsreaktion durchgeführt wird.

2. Verfahren zur Herstellung von Fettalkoholen durch Hydrierung der Methylester nach Anspruch 1, **dadurch gekennzeichnet**, daß das in der Hydrierreaktion der Methylester erzeugte unreine Methanol der Dampfreformierungsreaktion unterzogen wird und der auf diese Weise erhaltene Wasserstoff zum Anfang der Fettalkoholproduktionskette zurückgeführt wird.

3. Verfahren zur Herstellung von Fettalkohol durch Hydrierung der Methylester nach Anspruch 2, **dadurch gekennzeichnet**, daß man bei der Anwendung des Verfahrens einer Reformierung von unreinem Methanol, welches höhere Alkohole als Methanol enthält, in welchem das Wärmeangebot mittels eines wärmeführenden Fließmittels entsteht, das durch das Restgas einer Reinigung des durch Reformierung erzeugten Gases erhitzt wurde, zur Gewinnung von reinem Wasserstoff eine gleichzeitige Verbrennung des kondensierten Wasser-Alkoholgemisches, welches aus der Abkühlung des reformierten Gases stammt, einerseits und des aus der Wasserstoffreinigung stammenden Restgases durchführt.

4. Verfahren zur Reformierung von unreinem Methanol nach Anspruch 3, **dadurch gekennzeichnet**, daß nach einer geeigneten Ruhezeit das Gemisch von Wasser plus unreinem Methanol filtriert wird, um die feste Phase abzutrennen, und die filtrierte Flüssigkeit in die Reformiereinheit eingespeist wird.

5. Vorrichtung zur Herstellung von Fettalkoholen nach einem der Ansprüche 1 bis 4, **dadurch gekennzeichnet**, daß sie aus der Produktionskette A, die im wesentlichen einen Umesterungsreaktor (11 ) umfaßt, in welchem die Umsetzung von Methanol und der bei (12) und (13) eingeführten Fettmaterialien erfolgt, wobei die erzeugten Methylester durch die Leitung (15) entnommen werden, und dann der Produktionskette (B), die im wesentlichen einen Hydrierreaktor umfaßt, in welchen die Methylester (14) und der rückgeführte, aus der Dampfreformierung (17) stammende Wasserstoff (16) eingespeist werden, wobei die nach Trennung gebildeten Fettalkohole bei (18) entnommen werden und das unreine Methanol in der Leitung (19) zu der genannten Dampfreformierungh zirkuliert, besteht.

## Claims

1. Process for the production of fatty alcohols by the hydrogenation of methyl esters, characterised in that the hydrogenation reaction of methyl esters is carried out together with a methanol vapour reforming reaction.

2. Process for the production of fatty alcohols by the hydrogenation of methyl esters according to claim 1, characterised in that the impure methanol produced in the hydrogenation reaction of methyl esters is subjected to the vapour reforming reaction and the hydrogen thus obtained is recycled on entry to the fatty alcohols production line.

3. Process for the production of fatty alcohol by the hydrogenation of methyl esters according to claim 2, characterised in that, in the implementation of the process for reforming impure methanol containing higher alcohols than methanol, in which heat is supplied by means of a liquid coolant heated by the residual gas from a purification of the gas produced by reforming, for the purpose of obtaining pure hydrogen there is carried out simultaneous combustion of the condensed mixture of water + alcohols resulting from the cooling of reformed gas, on the one hand, and of residual gas resulting from the purification of hydrogen, on the other hand.

4. Process for the reforming of impure methanol according to claim 3, characterised in that, after an appropriate resting time, the mixture of water + impure methanol is filtered in order to separate out the solid phase, and the filtered liquid supplies the reforming unit.

5. Plant for the production of fatty alcohols according to any one of claims 1 to 4, characterised in that it is constituted by the production line A essentially comprising a transesterification reactor (11) in which there takes place the reaction of methanol and fatty substances introduced at (12) and (13), the methyl esters produced being drawn off by way of the circuit (14), and then by the production line B essentially comprising a hydrogenation reactor (15) supplied with methyl esters (14) and recycled hydrogen (16) resulting from the vapour reforming (17), the fatty alcohols formed after separation being drawn off at (18) and the impure methanol circulating in the pipe (19) towards the said vapour reforming.

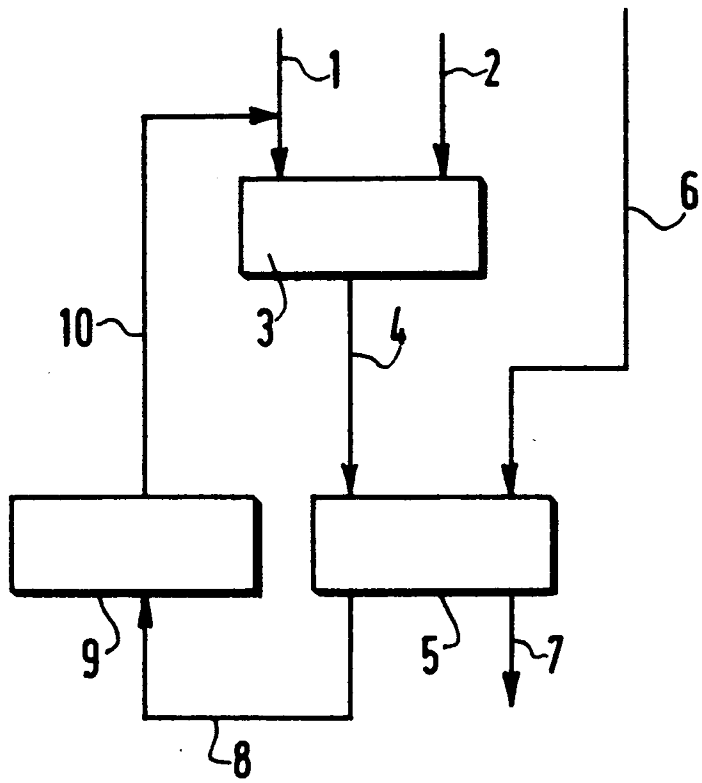


FIG.1

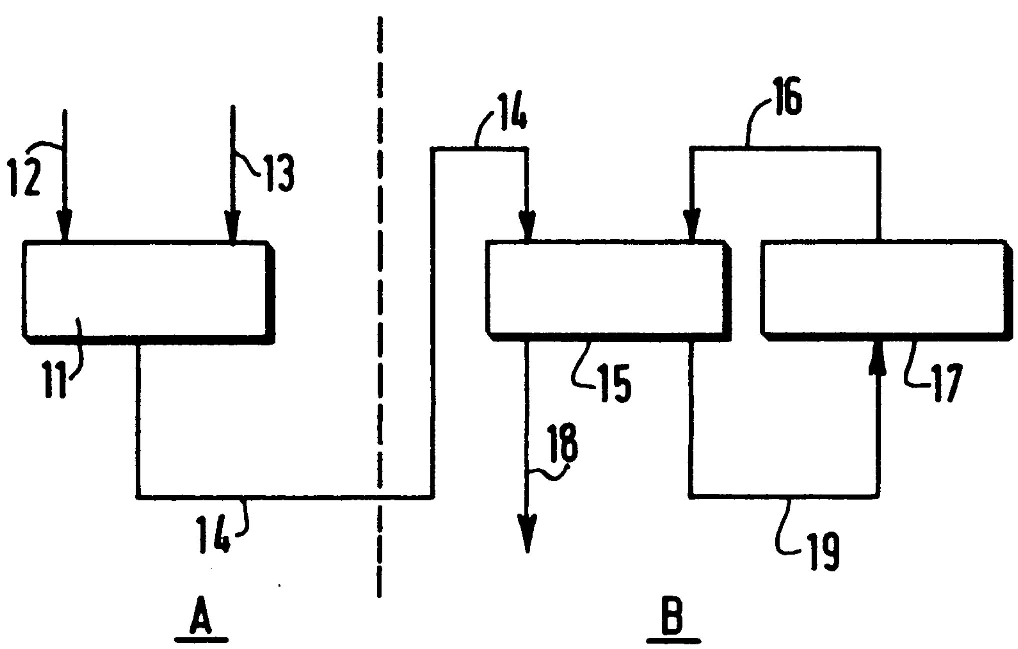


FIG.2